(19) Europäisches Patentamt
European Patent Office
Office européen des brevets

(11) Publication number: **0 149 297**
**B1**

(12) # EUROPEAN PATENT SPECIFICATION

(45) Date of publication of patent specification: **25.11.87**

(51) Int. Cl.[4]: **C 07 D 279/18**

(21) Application number: **84304476.9**

(22) Date of filing: **29.06.84**

(54) **Process for preparing 3H-Phenothlazin-3-ones.**

(30) Priority: **27.12.83 US 565269**

(43) Date of publication of application:
**24.07.85 Bulletin 85/30**

(45) Publication of the grant of the patent:
**25.11.87 Bulletin 87/48**

(84) Designated Contracting States:
**CH DE FR GB IT LI NL**

(56) References cited:
**DE-A-3 229 122**

**CHEMICAL ABSTRACTS, vol. 70, no. 1, January 6, 1969, Columbus, Ohio, USA, M. TERDIC "3H-Phenothiazin-3-ones. XI. Reaction of 2-aminothiophenols with halo-p-benzoquinones", page 375, abstract no. 3994h**

**CHEMICAL ABSTRACTS, vol. 58, no. 4, February 18, 1963, Columbus, Ohio, USA, C. BODEA "Phenothiazones VI. Preparation of 3-phenothiazone", column 3420, abstract no. 3420c**

(73) Proprietor: **MERCK FROSST CANADA INC.
16711 Trans-Canada Highway
Kirkland Quebec (CA)**

(72) Inventor: **Atkinson, Joseph G.
5643 Plantagenet Street
Montreal Quebec (CA)**

(74) Representative: **Crampton, Keith John Allen et al
D YOUNG & CO 10 Staple Inn
London WC1V 7RD (GB)**

**EP 0 149 297 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention involves the preparation of 3H-phenothiazin-3-ones.

The compound 3H-phenothiazin-3-one is known, as its preparation illustrated by the following equation:—

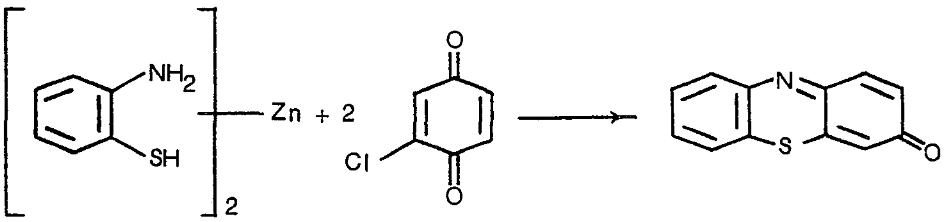

[see Terdic *et al.* Rev. Roum. de Chim, *13*, 834—836 (1968)].

The present invention provides a method for preparing 3H-phenothiazine-3-one comprising the reaction of 1 mole of *o*-mercaptoaniline with 2 moles of quinone in a polar solvent at a temperature in the range from −10°C to ambient.

This is illustrated by the following equation

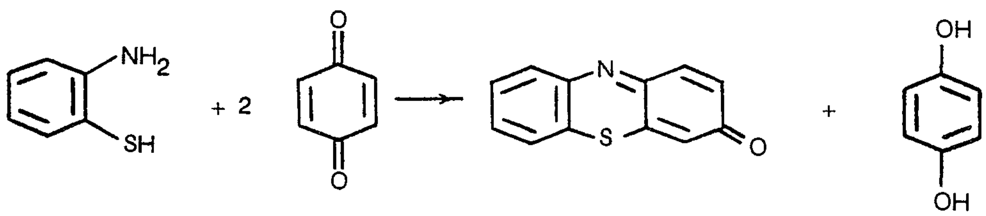

1 mol 2 mol

*A* *B* *C* *D*

The reaction requires 2 moles of quinone *B* per mole of aniline *A*. The reaction is readily carried out at room temperature, but temperatures as low as −10° are suitable. No catalyst or coupling agent is required. Any suitable polar solvent may be used. It is advantageous to use a solvent which will dissolve the *A, B* and *D* components of the reaction and in which *C* is substantially insoluble, thus permitting direct recovery of substantially pure *C*. Useful preferred solvents are lower alkanols, acetic acid, and mixtures of lower alkanols or acetic acid with water. Also in accordance with the present invention, there is provided a method for preparing 2,7-dimethoxy-3H-phenothiazin-3-one comprising the reaction of 1 mole of 2-amino-5-methoxy thiophenol with 2 moles of 2-methoxy-p-benzoquinone in a polar solvent at room temperature. This is carried out under similar conditions.

The following examples illustrate the present invention.

Example 1

3H-Phenothiazin-3-one

To a stirred suspension of 1.72 kg (16 mol) of quinone in 13 litres of methanol at room temperature was added slowly a solution of 1.0 kg (8 mol) of o-mercaptoamiline in 600 ml of methanol over a period of 1 hour. The resulting red mixture was stirred at room temperature for another 2 hours and then the 3H-phenothiazin-3-one was filtered off. This phenothiaz-3-one was washed thoroughly with methanol and dried to give 1.07 kg of 3H-phenothiazin-3-one (61.49% yield).

Example 2

2,7-Dimethoxy-3H-phenothiazin-3-one

Step 1: 2-Methoxy-quinone

Vanillin (2.432 kg) was added to a solution of sodium hydroxide (640 g) in water (8 l) and cooled to 10°C with an ice-bath. Then a solution of hydrogen peroxide (30%) (2.4 l) was added at a rate to keep the temperature of the action mixture below 30°C. After the addition had been completed (about 2 hours), the reaction mixture was added over a period of 3 hours to a suspension of sodium periodate (880 g) in water (4 l) and acetic acid (640 ml) cooled with an ice-bath to 10°C (the temperature of the reaction mixture was kept below 35°C). The precipitate was filtered, washed with cold water followed by ethanol/hexane (1:1) mixture and air-dried to afford the title compound (1.9 kg), m.p. 144—147°C.

Step 2: 2-Amino-5-methoxythiophenol

To a solution of 8N potassium hydroxide (1.3 l) was added 2-amino-6-methoxybenzothiazole (750 g) and the mixture was refluxed for 18 hours. The resulting solution was neutralized by the addition of

concentrated HCl, to pH 8.0, then acetic acid to pH 6.0. The precipitate which formed was filtered and washed with water to afford the title compound which was used immediately in Step 3.

Step 3: 2,7-Dimethoxy-3H-phenothiazin-3-one

To a suspension of 2-methoxy-quinone, (1.15 kg) (from Step 1) in methanol (8 l) was added portionwise a suspension of 2-amino-5-methoxythiophenol (from Step 2) in methanol (6 l). The reaction mixture was stirred for 15 minutes at room temperature and filtered, and the collected solid was washed with methanol (8 l). The product isolated was swished with dimethylformamide (DMF) (16 l) for 2 hours, filtered and air-dried. The crude material was dissolved in hot DMF (16 l) (130°C—140°C) and filtered through Celite (registered trade mark), and the filtrate was cooled to room temperature. The crystals were filtered, washed with methanol (8 l) and air-dried to afford the title compound (703 g), m.p. 237—283°C.

Some advantages of the present process over the prior process are that (1) it utilizes a simple readily available cheap quinone, namely, quinone itself or a derivative thereof, (2) it can be carried out at room temperature and yet afford a high yield and (3) the byproduct hydroquinone can be easily oxidized and recirculated for use in the process.

**Claims**

1. A method for preparing 3H-phenothiazine-3-one comprising the reaction of 1 mole of *o*-mercaptoaniline with 2 moles of quinone in a polar solvent at a temperature in the range from −10°C to ambient.

2. A method for preparing 2,7-dimethoxy-3H-phenothiazin-3-one comprising the reaction of 1 mole of 2-amino-5-methoxy thiophenol with 2 moles of 2-methoxy-p-benzoquinone in a polar solvent at room temperature.

3. A method as claimed in Claim 1 or 2 carried out at ambient temperature.

4. A method as claimed in any preceding claim in which the solvent is methanol, ethanol, acetic acid or a mixture of one or more of them with water.

**Patentansprüche**

1. Verfahren zur Herstellung von 3H-Phenothiazin-3-on durch Reaktion von 1 Mol o-Mercaptoanilin mit 2 Molen Chinon in einem polaren Lösungsmittel bei einer Temperatur im Bereich von −10°C bis Umgebungstemperatur.

2. Verfahren zur Herstellung von 2,7-Dimethoxy-3H-phenothiazin-3-on durch die Reaktion von 1 Mol 2-Amino-5-methoxythiophenol mit 2 Molen 2-Methoxy-p-benzochinon in einem polaren Lösungsmittel bei Raumtemperatur.

3. Verfahren, wie in Anspruch 1 oder 2 beansprucht, durchgeführt bei Umgebungstemperatur.

4. Verfahren, wie in einem der vorstehenden Ansprüche beansprucht, in welchem das Lösungsmittel Methanol, Ethanol, Essigsäure oder eine Mischung von einem oder mehreren davon mit Wasser ist.

**Revendications**

1. Procédé pour la préparation de la 3H-phénothiazine-3-one qui comprend la réaction d'une mole d'*o*-mercaptoaniline avec 2 moles de quinone, dans un solvant polaire à une température comprise dans la plage de −10°C à la température ambiante.

2. Procédé pour la préparation de la 2,7-diméthoxy-3H-phénothiazine-3-one qui comprend la réaction d'une mole de 2-amino-5-méthoxy-thiophénol avec 2 moles de 2-méthoxy-p-benzoquinone dans un solvant polaire à la température ambiante.

3. Procédé selon la revendication 1 ou 2, qui est effectué à la température ambiante.

4. Procédé selon l'une quelconque des revendications précédentes, dans lequel le solvant est le méthanol, l'éthanol, l'acide acétique ou un mélange d'un ou plusieurs d'entre eux avec l'eau.